# EUROPEAN PATENT APPLICATION

(11) **EP 1 201 303 A1**
(43) Date of publication of application: **02.05.2002**
(21) Application number: 00911407.5
(22) Date of filing: 28.03.2000
(51) Int. Cl.: B01J 39/20, B01J 31/08

(54) **CATIONIC EXCHANGE RESIN**

(30) Priority: 29.03.1999 JP 8700199; 30.03.1999 JP 8944099
(71) Applicant: Nippon Steel Chemical Co., Ltd., Tokyo 141-0031 (JP)
(72) Inventor: SAKURA, Katsuhiko, Nippon Steel Chemical Co., Ltd., Kitakyushu-shi, Fukuoka-ken 804-8503 (JP); UEDA, Shingo, Nippon Steel Chemical Co., Ltd., Kitakyushu-shi, Tukuoka-ken 804-8503 (JP); TAKEUCHI, Genki, Nippon Steel Chemical Co., Ltd., Oita-shi, Oita-ken 870-0111 (JP); KITOH, Taketoshi, Kitakyushu-shi, Fukuoka-ken 807-1111 (JP); KIMURA, Morio, Kitakyushu-shi, Fukuoka-ken 803-0835 (JP)
(74) Representative: Calamita, Roberto
(86) International application number: JP0001893
(87) International publication number: WO0058009

(57) **Abstract**

This invention relates to cationic exchange resins obtained by sulfonating copolymers of a monovinyl monomer mainly consisting of a styrenic monomer and a divinyl monomer wherein at least a part of the divinyl monomer is a polycyclic aromatic divinyl monomer, the degree of crosslinking expressed by (polycyclic aromatic divinyl monomer)/(total monomer) is 1 mol % or more, the exchange capacity is 0.7 meq/ml-wet resin or more, and the average crushing strength (Chatillon value) as determined on resin particles with an average diameter of 600 µm is 200 g/particle or more. Divinylbiphenyl is preferable as a polycyclic aromatic divinyl monomer. The cationic exchange resins exhibit high strength and activity and are useful as catalyst for the manufacture of bisphenol A from phenol and actone.

## Description

### Field of Technology

This invention relates to cationic exchange resins useful as catalyst.

### Background Technology

Cationic exchange resins are prepared by sulfonating copolymers of a monovinymonomer such as styrene and a divinyl monomer such as divinylbenzene is most commonly used as a divinyl monomer. A divinyl monomer acts as a crosslinking agent and an increase in its proportion greatly affects the properties of copolymers by increasing strength and brittleness and decreasing water content and degree of swelling. In particular, the performance as catalyst is greatly affected.

For example, as indicated in the specification of Japan Patent JP06-32755 A (1994), an increasing proportion of a divinyl monomer as a crosslinking agent greatly affects the catalytic performance of cationic exchange resins, the catalytic activity tending to diminish with the passage of time for one thing.

An approach is known which changes not only the proportion but also the kind of a divinyl monomer (JP02-298357A(1990) and elsewhere), but few surpass divinylbenzene. Moreover, a large number of patents and literature teach the use of cationic exchange resins as catalyst in the manufacture of bisphenol A from acetone and phenol.

An object of this invention is to provide cationic exchange resins which contain sulfonic acid group introduced at a high rate relative to the degree of crosslinking and hence exhibit a good balance of properties as ion exchange resins. Another object of this invention is to provide high-strength cationic exchange resins.

A further object of this invention is to provide acid catalysts of high activity on cationic exchange resins. A still further object of this invention is to provide catalysts based on cationic exchange resins which are not only highly active but also capable of maintaining the activity over a long period of time.

### Disclosure of the Invention

This invention relates to cationic exchange resins obtained by sulfonating copolymers of a monovinyl monomer mainly consisting of a styrenic monomer and a divinyl monomer wherein at least a part of the divinyl monomer is a polycyclic aromatic divinyl monomer, the degree of crosslinking expressed by (polycyclic aromatic divinyl monomer)/(total monomer) is 1 mol % or more, the exchange capacity is 0.7 meq/ml·wet resin or more, and the average crushing strength (Chatillon value) measured on particles with a diameter of 600 µm is 200 g/particle or more. It is preferable here that the degree of crosslinking expressed by (polycyclic aromatic divinyl monomer)/(total monomer) is 2 mol % or more.

Moreover, this invention relates to the aforementioned cationic exchange resin wherein the degree of crosslinking expressed by (polycyclic aromatic divinyl monomer)/(total monomer) is 5 mol % or less, the exchange capacity is 2.5 meq/ml-wet resin or less, and the water content under the air-dried condition is 60 wt % or more. It is preferable here that the degree of crosslinking expressed by (polycyclic aromatic divinyl monomer)/(total monomer) is 1-4 mol % and the exchange capacity is 0.6-1 meq/ml-wet resin. Divinylbiphenyl is preferable as a polycyclic aromatic divinyl monomer.

This invention further relates to catalysts comprising the aforementioned cationic exchange resins. Still further, this invention relates to the aforementioned catalysts for the manufacture of bisphenol A from phenol and acetone.

The aforementioned divinyl monomer needs at least in part to be a polycyclic aromatic divinyl monomer and the proportion thereof is preferably 20 mol % or more preferably 50 mol % or more, most preferably 70 mol % or more. The rest of the divinyl monomer can be a monocyclic monomer such as divinylbenzene, preferably divinylbenzene. The higher the proportion of a polycyclic aromatic divinyl monomer, the higher the strength of cationic exchange resins; cationic exchange resins of higher strength are less prone to undergo elastic deformation when use while packed in a fixed bed and the pressure loss in the packed layer can be reduced. In case divinylbenzene is used as a divinyl monomer, the strength increasas the degree of crosslinking increases, but so does the brittleness disadvantageously. However, the use of a polycyclic aromatic vinyl monomer as a divinyl monomer is characterized by that the strength is increased by increasing the degree of crosslinking while leaving the brittleness unaffected. Moreover, in the case of cationic exchange resins, an increasing ratio of the polycyclic aromatic divinyl monomer to divinylbenzene increases the distance between crosslinks and relaxes the steric hindrance around the aromatic ring, which allows the sulfonation by sulfuric acid to proceed to a greater extent with the resultant high exchange capacity. Moreover, the steric hindrance around the sulfonic acid group that is an active site of the catalyst is also relaxed and cationic exchange resins, when used as catalyst, exhibit a high catalytic activity. A phenomenon such as this also occurs in ion exchange resins other than those cationic exchange resins which involve the introduction of sulfonic acid group by sulfuric acid during their preparation. Such other ion exchange resins are, for example, anion exchange resins prepared by chloromethylating copolymers of styrene and a divinymonomer followed by treating with an amine such as trimethylamine and chelate resins prepared by introducing to the aromatic ring a functional group capable of forming a chelate with a metal; in these resins, an increasing ratio of a polcyclic aromatic divinyl monomer to divinylbenzene increases the distance between crosslinks with the resultant higher exchange capacity.

Moreover, cationic exchange resins when used as catalyst exhibit a higher reactivity and maintain the reactivity over a longer period of time as the degree of crosslinking expressed by (polycyclic aromatic divinyl monomer)/(total monomer) increases even when the ratio (total divinyl monomer)/(total monomer) remains the same.

Cationic exchange resins are known to show a higher exchange capacity under wet condition as the ratio (total divinyl monomer)/(total monomer) increases. High exchange capacity is a property generally desired for cationic exchange resins, but when the resins are used as catalyst, an exchange capacity which is higher than is necessary rather causes a decrease not only in catalytic activity but also longterm catalytic activity. However, the present inventors have found that, in case the ratio (total divinyl monomer)/(total monomer) was increased by in - creasing the proportion of a polycyclic aromatic divinyl monomer to be used as a divinyl monomer, the catalytic activity increased as the exchange capacity increased and the activity was maintained over a long period of time. It is generally observed in the case of cationic exchange resins that an increasing ratio of the polycyclic aromatic divinyl monomer to divinylbenzene increases the distance between crosslinks and relaxes the steric hindrance around the aromatic ring, allowing the sulfonation by sulfuric acid to proceed to a greater extent with the resultant higher exchange capacity. Moreover, the steric hindrance around the sulfonic acid group that is an active site of the catalyst is also relaxed and cationic exchange resins, when used as catalyst, exhibit a high catalytic activity. As mentioned above, a phenomenon such as this also occurs in ion exchange resins other than cationic exchange resins involving the introduction of sulfonic acid group by sulfuric acid.

Polycyclic aromatic divinyl monomers include compounds containing two vinyl groups derived from condensed aromatic hydrocarbons such as naphthalene, anthracene, and phenanthrene, noncondensed aromatic hydrocarbons composed of directly linked aromatic rings such as biphenyl and terphenyl, and aliphatic hydrocarbons substituted with two or more aromatic rings such as diphenylmethane and diphenylethane. The number of aromatic rings is 2 or more, preferably 2 or 3. The divinyl groups are preferably substituted on different rings, particularly at symmetrical positions or at positions adjacent to symmetrical positions. Moreover, the distance between the vinyl groups ranges from 1.5 to 3 times that in divinylbenzene. Suitable divinyl monomers include those materials which mainly contain 4,4'-divinylbiphenyl, 3,4'-divinylbiphenyl, 3,3'-divinylbiphenyl, 2,6-divinyl naphthalene, and 2,7-divinylnaphthalene. The raw material divinyl monomer may be a pure material or it may contain partially dehydrogenated material, that is, a polycyclic aromatic compound containing a vinyl group and an ethyl group without ill effect. Rather, it is preferable for the raw material to contain 4-40 wt % of such polycyclic aromatic compound containing a vinyl group and an ethyl group. Such compound is naturally computed as a monovinyl monomer.

The degree of crosslinking expressed by (polycyclic aromatic divinyl monomer)/(total monomer) is 1 mol % or more, preferably 2 mol % or more. It is avantageously 2-40 mol %, more advantageously 2-20 mol %, most advantageously 3-10mol %. The degree of total crosslinking expressed by (total divinyl monomer)/(total monomer) is 2-50 mol %, preferably 2-20 mol %, more preferably 3-10 mol %. The strength and catalytic performance deteriorate as the degree of total crosslinking diminishes.

Ion exchange resins with a degree of crosslinking expressed by (polycyclic aromatic divinyl monomer)/(total monomer) of 5 mol % or less are desirable because such resins exhibit an improved life when used as catalyst. In this case, the range of the aforementioned degree of crosslinking is 1-4 mol %, preferably 2-4 mol %,more preferably 2-3 mol %. The degree of crosslinking expressed by (total divinymonomer)/(total monomer) is 1-30 mol %, preferably 1-5 mol %. The strength increases, but the catalyst life may decrease when this degree of crosslinking is high.

In an overall consideration of the strength, catalytic performance, and catalyst life, the preferable range of the aforementioned degree of crosslinking is 1-10 mol %, more preferably 2-7 mol %, most preferably 3-5 mol %. In this case, the degree of crosslinking expressed by (total divinyl monomer)/(total monomer) is 1-10 mol %, preferably 2-7 mol %, more preferably 3-5 mol %. An increase in this degree of crosslinking increases the strength of ion exchange resin, but decrease the catalyst life.

The copolymerization of a divinyl monomer and a monovinyl monomer can be effected in accordance with a known method and copolymers of gel type, porous type or microporous type can be obtained by controlling the polymerization conditions and the degree of crosslinking. Although cationic exchange resins of this invention may be any of the gel type, porous type, and microporous type, the use of the gel type is preferable when the degree of crosslinking expressed by (poly - cyclic aromatic divinyl polymer)/(total monomer) is 1-30 mol %, preferably 2-10 mol % while the use of the porous or microporous type is preferable when the degree of crosslinking is 10 mol % or more.

The copolymers can be sulfonated by a known method. The degree of introduction of the sulfonic acid group to the benzene ring varies with the degree of crosslinking, but it is 0.90 or more, preferably 0.92-0.98.

Cationic exchange resins of this invention thus prepared preferably exhibit an exchange capacity of 0.7 meq/ml-wet resin or more and an average crushing strength (Chatillon value) of 200 g/particle or more when measured on particles with an average diameter of 600 µm.

High exchange capacity is a property generally desired for cationic exchange resins and the cationic exchange efficiency per unit volume of ion exchange resin improves as the value of this property increases. Cationic exchange resin with a high exchange capacity exhibits an improved activity as acid catalyst and the exchange capacity is at least 0.7 meq/ml-wet resin or more, preferably 1.0 meq/ml-wet resin.

In consideration of the activity and life of cationic exchange resins as catalyst, the exchange capacity of cationic exchange resins of this invention is advantageously 2.5 meq/ml-wet resin or less. Although high exchange capacity is desirable for cationic exchange resins in general, consideration of the activity and life of cationic exchange resins as catalyst suggests that the exchange capacity falls in the range 0.5-2.0 meq/ml-wet resin, preferably 0.7-1.5 meq/ml-wet resin. Furthermore, the water content under air-dried condition needs to be 48% or more. As the water content is related to the activity and catalyst life, it would be better to control it at 50% or more, preferably 60% or more.

The crushing strength (Chatillon value) of ion exchange resin particles is desirably as high as possible in practical use and is at least 200 g/particle or more, preferably 300 g/particle or more. When filled in a fixed bed and used as catalyst, ion exchange resin with a crushing strength lower than the above develops the possibility of the resin undergoing elastic deformation and the resin-filled layer suffering an increase in pressure loss. When used as a reaction catalyst, there is the possiblity of the activity diminishing.

Cationic exchange resins of this invention are used effectively in water treatment such as waste water treatment, recovery of valuable metals, and soften of hard water and in the production of pure water for use in boilers at thermal and nuclear power plants. They are also used effectively in such processes as purification of medical fluids, refining of sugar liquor, separation and purification of amino acids, and purification of shochu (alcoholic beverage) in the pharmaceutical and food industries. In addition to the aforementioned, cationic exchange resins are used widely as catalysts in the manufacture of a variety of industrial products. Cationic exchange resins of this invention characterized by containing a polycyclic aromatic divinyl monomer as part of structural units are superior to the conventional cationic exchange resins containing only a monocyclo aromatic divinyl monomer as structural unit in respect to strength, ion exchange efficiency, and catalytic activity and are applied more effectively to the aforementioned uses.

Bisphenol A is prepared by the reaction of phenol with acetone in the presence of cationic exchange resin as catalyst and the catalyst for this reaction desirably exhibits high activity, maintains a long life, and causes a low pressure loss in the reactor. Cationic exchange resins of this invention give an excellent performance as catalyst for the manufacture of bisphenol A.

In case cationic exchange resin of this invention is used as a catalyst for the manufacture of bisphenol A, a fixed-bed reactor is filled with the resin and the reaction is carried out under the following conditions: LHSV, 0.1-20 hr ⁻¹, preferably 0.3-6 hr ⁻¹; temperature, 45-140 °C, preferably 55-100 °C; and molar ratio of acetone to phenol, 0.005-0.5, preferably 0.01-0.3. If necessary, a thiols such as mercaptan, mercaptoethanol, and mercaptopropionic acid is added as a reaction accelerator in an amount of 0.01-2 wt %.

### Preferred Embodiments of the Invention

### Example 1

In a 500-ml three-necked separable flask fitted with a stirrer and a condenser was placed 235 g of water and 0.1 g of polyvinyl alcohol and then 44.1 g of styrene, 5.9 g of a solution of crosslinking agents composed of 52% divinyl biphenyl (predominantly 4,4'-divinylbiphenyl, % is wt %), 30% ethylvinylbiphenyl and 18% vinylbiphenyl, and 0.1 g of benzoyl peroxide were added. The mixture was allowed to react at 80°C for 10 hours with stirring while introducing nitrogen gas into the system. The copolymers were recovered and air-dried and they were swollen by placing 30 g of the air-dried copolymers in a 300-ml four-necked flask adding 60 g of water and 150 g of nitrobenzene, and stirring the mixture at 70°C for 2 hours.

The swollen copolymers were recovered, air-dried, placed wholly in a 300-ml four necked flask, and stirred with 150 g of concentrated sulfuric acid at 80 °C for 10 hours to effect sulfonation. After the reaction, the copolymers were collected by filtration and washed with 1,200 g of water to give cationic exchange resin.

The resin thus obtained exhibited a degree of crosslinking of 3.3 mol %, an exchange capacity of 1.31 meq/ml-wet resin, and a Chatillon value of 440 g/particles determined by the crushing test conducted on 100 resin particles with a diameter of 600 µm.

### Examples 2-5

Cationic exchange resins were prepared as in Example 1 except substituting a part of divinylbiphenyl for divinylbenzene.

### Comparative Example 1

Cationic exchange resin was prepared as in Example 1 except substituting divinylbiphenyl in whole for divinylbenzene. The divinylbenzene in solution was composed of 57% divinylbenzene and 43% ethylvinylbenzene.

The cationic exchange resins prepared in Examples 1-5 and Comparative Examplel were tested for their properties and their performance as catalyst in the manufacture of bisphenol A. The results are shown in Table 1. The degree of crosslinking was 3.3 mol %, 3.0 mol %, 2.6 mol %, 2.0 mol %, 1.3 mol %, and 0.7 mol % in the order of Examples 1 to 5 and Comparative Example 1 while the degree of total crosslinking expressed by (total divinyl monomer)/(total monomer) was 3.3 mol % in any of the examples and comparative example. The reaction was carried out as follows.

Phenol was allowed to react with acetone in the presence of ethylmercaptan as a reaction accelerator to give a reaction mixture containing bisphenol A. The reaction mixture was distilled to remove low-boiling substances such as acetone, water, and ethylmercaptan, the bisphenol A-phenol adduct was crystallized and filtered off to give the mother liquor (containing 85% phenol, 8% bisphenol A, 5% 2,4-isomer, and 2% other impurities). A mixture of 1,000 parts by weight of the mother liquor, 30 parts by weight of acetone, and 2 parts by weight of ethylmercaptan as feedstock was introduced continuously at a rate of 50 ml/hr at 70°C to a flow type reactor which is made of stainless steel, 1 cm in inside diameter, and filled with 50 ml of the cationic exchange resin respectively prepared in Examples 1-5 and Comparative Example 1. The reaction was continued for 150 day and the conversion of acetone and the pressure loss after passage of 150 days were determined. The drop in conversion in Table 1 means the difference in conversion of acetone between the 0th day and 150th day.

**Table 1**

| Ex. | DVBP/DVB (molar ratio) | Chatillon value (g/particle) | Water content (%) | Exchange capacity (meq/ml-resin) | Conversion of acetone (%) | | | Pressure loss (kg/cm²) |
|---|---|---|---|---|---|---|---|---|
| | | | | | 0th day | 150th day | Drop | |
| 1 | 10/0 | 440 | 64.4 | 1.31 | 93.6 | 88.5 | 5.1 | 4.5 |
| 2 | 9/1 | 430 | 64.5 | 1.30 | 93.5 | 84.7 | 8.8 | 4.5 |
| 3 | 8/2 | 430 | 64.7 | 1.29 | 93.4 | 81.4 | 12.0 | 4.6 |
| 4 | 4/6 | 430 | 65.1 | 1.28 | 91.1 | 66.5 | 24.6 | 4.8 |
| 5 | 2/8 | 420 | 65.4 | 1.27 | 90.5 | 59.1 | 31.4 | 5.0 |
| comp. ex. 1 | 0/10 | 380 | 66.0 | 1.25 | 89.7 | 52.4 | 37.3 | 5.5 |

### Examples 6-14

In experiments carried out as in Example 1, nine kinds of cationic exchange resins differing from one another in the degree of crosslinking were prepared by changing the ratio of styrene to divinylbiphenyl.

### Examples 15-18

Four kinds of cationic exchange resins differing from one another in the degree of crosslinking were prepared as in Example 1 except using a solution of divinylnaphthalene (containing 34% 2,6-divinylnaphthalene, 38% 2,7-divinylnaphthalene, and 18% ethyl(vinyl)naphthalene, the remainder being diethylnaphthalene and others) as a solution of crosslinking agents and changing the ratio of styrene to dvinylnaphthalene.

### Comparative Examples 2-5

Four kinds of cationic exchange resins differing from one another in the degree of crosslinking were prepared as in Example 1 from commercially available styrenedivinylbenzene copolymers (gel type).

The cationic exchange resins prepared in Examples 6-18 and Comparative Examples 2-5 were measured for selected properties and the results are shown in Table 2.

The reaction for the manufacture of bisphenol A from phenol and acetone was carried out in the presence of each of the cationic exchange resins of Examples 6-18 and Comparative Examples 2-5 as catalyst. The raw material consisting of phenol and acetone at a weight ratio of 11.25 and containing 0.2 wt % of ethylmecaptan was mixed with 6 ml of wet cationic exchange resin and the reaction was carried out at 70 °C for 2 hours and the conversion of the acetone was measured. The results are shown in Table 2.

**Table 2**

| Example No. | Degree of cross-linking mol % | Degree of total cross-linking mol % | Exchange capacity meq/ml | Density g/ml-wt resin | Chatillon value g/particle | Water content % | Sulfonic acid group/ benzene ring | Conversion of acetone % |
|---|---|---|---|---|---|---|---|---|
| 6 | 1.6 | 1.6 | 0.79 | 0.605 | 180 | 78.4 | 0.95 | 60.0 |
| 7 | 2.0 | 2.0 | 0.99 | 0.620 | 220 | 73.3 | 0.95 | 67.2 |
| 8 | 2.4 | 2.4 | 1.03 | 0.639 | 290 | 70.6 | 0.94 | 70.5 |
| 9 | 2.7 | 2.7 | 1.13 | 0.671 | 350 | 71.7 | 0.93 | 71.3 |
| 10 | 3.2 | 3.2 | 1.29 | 0.714 | 440 | 65.4 | 0.92 | 72.4 |
| 11 | 4.0 | 4.0 | 1.48 | 0.726 | 480 | 60.7 | 0.92 | 72.5 |
| 12 | 4.5 | 4.5 | 1.68 | 0.749 | 520 | 58.8 | 0.92 | 74.1 |
| 13 | 5.0 | 5.0 | 1.77 | 0.765 | 550 | 53.6 | 0.92 | 73.7 |
| 14 | 6.7 | 6.7 | 2.21 | 0.785 | 570 | 44.3 | 0.90 | 72.1 |
| 15 | 2.4 | 2.4 | 1.24 | 0.685 | 310 | 68.9 | 0.99 | 74.3 |
| 16 | 3.2 | 3.2 | 1.51 | 0.720 | 450 | 63.0 | 0.99 | 75.5 |
| 17 | 4.0 | 4.0 | 1.69 | 0.729 | 490 | 59.2 | 0.98 | 75.7 |
| 18 | 6.7 | 6.7 | 2.36 | 0.807 | 610 | 41.1 | 0.95 | 74.9 |
| Comp. ex.2 | 0 | 2.4 | 0.99 | 0.697 | 290 | 73.2 | 0.94 | 55.3 |
| 3 | 0 | 2.7 | 1.11 | 0.722 | 350 | 69.8 | 0.91 | 65.9 |
| 4 | 0 | 3.2 | 1.23 | 0.748 | 380 | 66.6 | 0.90 | 69.3 |
| 5 | 0 | 4.0 | 1.45 | 0.759 | 430 | 61.0 | 0.89 | 40.3 |

### Example 19 and Comparative Example 6

The reaction of phenol with acetone for the manufacture of bisphenol A was caried out in the following manner in the presence of the catalyst respectively using the aforementioned Examples 6-18 and Comparative Examples 2-5.

The reaction mixture resulting from the reaction of phenol with acetone in the presence of ethylmercaptan as a reaction accelerator was distilled to remove low-boiling substances such as acetone, water, and ethylmercaptan, the bisphenolA-phenol adduct was crystallized out, and the separated crystals were filtered off to obtain the mother liquor (containing 85% phenol, 8% bisphenol A, 5% 2,4-isomer, and 2% other impurities). A mixture of 1,000 parts by weight of the mother liquor, 30 parts by weight of acetone, and 2 parts by weight of ethylmercaptan was introduced continuously at a rate of 50 ml/hr and at 70°C to a flow type reactor that is made of stainless steel, 1 cm in inside diameter, and filled with 50 ml of the aforementioned cationic exchange resin as catalyst. The reaction was continued for 150 days and the conversion of the acetone was determined.

The results are shown in Table 3. The number in the catalyst (Example No.) corresponds to the Example No. in Table 2, that is, the catalyst used here is the same as the one used in the corresponding Example No.

**Table 3**

| Catalyst (Example No.) | Degree of crosslinking mol % | Degree of total crosslinking mol% | Conversion of acetone, % | | |
|---|---|---|---|---|---|
| | | | 0th day | 150th day | Drop |
| 6 | 1.6 | 1.6 | 85.9 | 84.1 | 1.8 |
| 7 | 2.0 | 2.0 | 88.1 | 86.1 | 2.0 |
| 8 | 2.4 | 2.4 | 90.3 | 88.0 | 2.3 |
| 9 | 2.7 | 2.7 | 92.5 | 88.3 | 4.2 |
| 10 | 3.2 | 3.2 | 93.0 | 88.7 | 4.3 |
| 11 | 4.0 | 4.0 | 93.6 | 89.2 | 4.4 |
| 12 | 4.5 | 4.5 | 95.3 | 89.6 | 5.7 |
| 13 | 5.0 | 5.0 | 94.7 | 88.7 | 6.0 |
| 14 | 6.7 | 6.7 | 93.5 | 84.6 | 8.9 |
| 15 | 2.4 | 2.4 | 95.6 | 94.2 | 1.4 |
| 16 | 3.2 | 3.2 | 96.8 | 94.3 | 2.5 |
| 17 | 4.0 | 4.0 | 97.0 | 93.2 | 3.8 |
| 18 | 6.7 | 6.7 | 96.0 | 87.6 | 8.4 |
| Comp. Ex. 2 | 0 | 2.4 | 80.3 | 65.8 | 14.5 |
| 3 | 0 | 2.7 | 87.5 | 55.6 | 31.9 |
| 4 | 0 | 3.2 | 89.5 | 52.6 | 36.9 |
| 5 | 0 | 4.0 | 71.6 | 31.4 | 40.2 |

### Industrial Applicability

Cationic exchange resins of this invention exhibit a high content of the introduced sulfonic acid group relative to the degree of crosslinking and are well balanced in the properties of ion exchange resins. Cationic exchange resins of this invention exhibit not only a high strength but also good activity when used as catalyst. In consequence, they are useful as catalyst for the manufacture of bisphenol A from phenol and acetone.

## Claims

1. Cationic exchange resins obtained by sulfonating copolymers of a monovinyl monomer mainly comprising of a styrenic monomer and a divinyl monomer wherein at least a part of the divinyl monomer is a polycyclic aromatic divinyl monomer, the degree of crosslinking expressed by (polycyclic aromatic divinyl monomer)/(total monomer) is 1 mol % or more, the exchange capacity is 0.7 meq/ml-wet resin or more and the average crushing strength (Chatillon value) as determined on resin particles with an average diameter of 600 µm is 200 g/particle or more.

2. Cationic exchange resins as described in claim 1 wherein the degree of crosslinking expressed by (polycyclic aromatic divinyl monomer)/(total monomer) is 2 mol % or more.

3. Cationic exchange resins as described in claim 1 wherein the degree of crosslinking expressed by (polycyclic aromatic divinyl monomer)/(total monomer) is 5 mol % or less, the exchange capacity is 2.5 meq/ml-wet resin or less, and the water content under air-dried condition is 60 wt % or more.

4. Cationic exchange resins as described in claim 3 wherein the degree of crosslinking expressed by (polycyclic aromatic divinyl monomer)/(total monomer) is 1-4 mol % and the exchange capacity is 0.6-1 meq/ml-wet resin.

5. Cationic exchange resins as described in claim 1 wherein the polycyclic aromatic divinyl monomer is divinylbiphenyl.

6. Catalysts based on cationic exchange resins comprising cationic exchange resins described in any one of claims 1 to 5.

7. Catalyst as described in claim 6 is a catalyst for the manufacture of bisphenol A from phenol and acetone.
